# EUROPÄISCHE PATENTANMELDUNG

(11) **EP 2 597 144 A1**
(43) Veröffentlichungstag der Anmeldung: **29.05.2013**
(21) Anmeldenummer: 12194062.1
(22) Anmeldetag: 23.11.2012
(51) Int. Cl.: C12M 1/107

(54) **Vorrichtung zur Fermentation organischer Feststoffe**

(30) Priorität: 24.11.2011 DE 102011055695
(71) Anmelder: Renergon International AG, 8280 Kreuzlingen (CH)
(72) Erfinder: Restle, Karl-Heinz, 8280 Kreuzlingen (CH); Betz, Gebhard, 89597 Munderkingen (DE)
(74) Vertreter: Nordmeyer, Philipp Werner

(57) **Zusammenfassung**

Die vorliegende Erfindung betrifft eine Vorrichtung (1) zur Fermentation organischer Feststoffe, umfassend mindestens eine Fermentationskammer (2) zum Fermentieren organischer Feststoffe und mindestens eine Perkolationsvorrichtung (3) zum Aufbringen von Perkolat auf organische Feststoffe in der Fermentationskammer (2), wobei mindestens ein Behälter (4) zum Aufnehmen der organischen Feststoffe in der Fermentationskammer (2) vorgesehen ist. Weiterhin ist ein Verfahren zur Fermentation organischer Feststoffe angegeben, umfassend das Einbringen des organischen Feststoffs in eine Fermentationskammer (2) und das Aufbringen von Perkolat auf den in die Fermentationskammer (2) eingebrachten organischen Feststoff, wobei der organische Feststoff in mindestens einem Behälter (4) in die Fermentationskammer eingebracht wird und in diesem Behälter (4) in der Fermentationskammer fermentiert wird.

## Beschreibung

### Technisches Gebiet

Die vorliegende Erfindung betrifft eine Vorrichtung zur Fermentation organischer Feststoffe, bevorzugt zur Fermentation stapelbarer Biomasse wie beispielsweise Biomüll, biogene Abfallstoffe, Ernterückstände, Landschaftspflegegrün und/oder nachwachsende Rohstoffe.

### Stand der Technik

Es ist bekannt, organische Feststoffe und insbesondere stapelbare Biomasse in so genannten Feststofffermentern zu fermentieren, um hieraus Biogas zu gewinnen. Das Biogas wird nachfolgend thermisch zur Wärmeerzeugung und/oder über Blockheizkraftwerke zur gekoppelten Strom- und Wärmegewinnung verwertet.

In den bekannten Feststofffermentern wird die Biomasse in eine Fermentationskammer eingebracht und dann periodisch von einem Perkolatstrom durchströmt. In dem bekannten Verfahren wird die Biomasse in einem anaeroben Milieu vergoren, wobei die entsprechenden Bakterien mittels des Perkolats zugeführt werden und die Biomasse entsprechend "geimpft" wird. Die Biomasse wird dann unter Bildung von Biogas zersetzt, wobei der entsprechende Vergärungsprozess in mehreren aufeinanderfolgenden Stufen verläuft. Dieser Prozess kann entsprechend in die folgenden Phasen unterteilt werden: Hydrolyse, Acidogenese, Acetogenese und Methanogenese. Das daraus entstehende Biogas setzt sich zum großen Teil aus Methan zusammen. Es handelt sich hierbei entsprechend um ein energiereiches Gas, welches unter den anoxischen Bedingungen aus den organischen Stoffen entsteht.

In den bekannten Anlagen zur Fermentation organischer Feststoffe werden die organischen Feststoffe in die jeweiligen Fermentationskammern mittels Förderbändern oder schwerem fahrbarem Gerät, wie beispielsweise Baggern bzw. Radladern, eingebracht. Die Biomasse liegt dann auf dem Boden der jeweiligen Fermentationskammer und wird entsprechend auch entlang der Wände aufgeschüttet.

Der Perkolatstrom wird von der Decke der Fermentationskammer aus auf die Biomasse aufgebracht, um den Gärprozess zu starten und in Gang zu halten.

Die entsprechenden Fermentationskammern sind gasdicht mittels schwerer Türen verschlossen und das Biogas wird über entsprechende Leitungsvorrichtungen aus den Fermentationskammern abgezogen.

Nachdem der Vergärprozess abgeschlossen ist, also ungefähr nach 4 Wochen, wird der Gärrest wieder aus den Fermentationskammern ausgetragen, wobei dies wiederum entweder mittels schwerem Gerät, wie beispielsweise Radladern oder anderen Baggern oder mit Förderbändern oder anderen Fördervorrichtungen vorgenommen wird.

Die bekannten Fermentationsanlagen sind für große Mengen an Biomasse und bei bestehender Infrastruktur sehr gut geeignet.

Wenn jedoch Insellösungen aufgebaut werden sollen, welche über keine oder nur sehr eingeschränkte Infrastruktur verfügen, beispielsweise in schwer zugänglichen Gebieten, ist der Einsatz von schwerem Gerät zum Beschicken der Fermentationskammern schwierig oder ungeeignet. Weiterhin sind die massiven Strukturen der Fermentationskammern, so wie sie im Stand der Technik bekannt sind, bei einer Insellösung nur schwer bereitzustellen.

### Darstellung der Erfindung

Entsprechend ist es eine Aufgabe der vorliegenden Erfindung, eine Vorrichtung zur Fermentation organischer Feststoffe anzugeben, welche auch vorteilhaft als Insellösung ohne aufwändige Infrastruktur betrieben werden kann.

Diese Aufgabe wird mittels einer Vorrichtung zur Fermentation organischer Feststoffe mit den Merkmalen des Anspruchs 1 gelöst. Vorteilhafte Ausgestaltungen ergeben sich aus den Unteransprüchen.

Entsprechend ist eine Vorrichtung zur Fermentation organischer Feststoffe vorgesehen, welche mindestens eine Fermentationskammer zum Fermentieren der organischen Feststoffe und mindestens eine Perkolationsvorrichtung zum Aufbringen von Perkolat auf die organischen Feststoffe in der Fermentationskammer umfasst. Erfindungsgemäß ist mindestens ein Behälter zum Aufnehmen der organischen Feststoffe in der Fermentationskammer vorgesehen.

Durch die Fermentation wird dann das Biogas gewonnen, welches nachfolgend beispielsweise zur direkten Verbrennung oder zum Betrieb eines Blockheizkraftwerkes verwendet wird.

Dadurch, dass die organischen Feststoffe in dem Behälter aufgenommen werden und die organischen Feststoffe dann entsprechend in dem Behälter in die Fermentationskammer eingebracht werden, ist es möglich, die Handhabung der organischen Feststoffe beim Einbringen bzw. beim Austragen des Gärrestes deutlich zu vereinfachen. Insbesondere kann über eine geeignete Wahl der Behältergröße erreicht werden, dass die Behälter einfach manuell von den entsprechenden Bedienern der Vorrichtung in die Fermentationskammer eingestellt bzw. aus dieser wieder entnommen werden können.

Darüber hinaus kann durch die Verwendung des Behälters zur Aufnahme des organischen Feststoffes der gesamte Prozess der Handhabung des organischen Feststoffes vom Einsammeln bis hin zum Wiederverwenden bzw. Verwerten des Gärrestes, beispielsweise als Dünger, deutlich vereinfacht werden. Beispielsweise kann in abgelegenen Gebieten, in welchen eine entsprechende Infrastruktur nicht oder nur spärlich vorhanden ist, der entsprechende Abfall bereits in den jeweiligen Behältern gesammelt werden, welche dann vollständig, sobald sie gefüllt sind, in die jeweilige Fermentationskammer eingestellt werden. Hierzu sind die Behälter vorzugsweise stapelbar, um eine hohe Raumausnutzung in der Fermentationskammer zu ermöglichen, wobei gleichzeitig die Handhabung durch entsprechend kleinere Behälter vereinfacht wird.

Die Vorteile der Vereinfachung in der Handhabung der organischen Feststoffe sowie der Handhabung des Gärrests überwiegen in dem angegebenen Anwendungsbereich die Nachteile, die sich dadurch ergeben, dass ein beträchtlicher Teil des Volumens der Fermentationskammer mit dem Materialvolumen der Behälters gefüllt wird. Es ist nachzuvollziehen, dass die Behälter gerade nicht aus einem fermentierbaren Material bestehen sollen, um die Handhabung des organischen Materials sowie des Gärrestes zu gewährleisten. Entsprechend trägt das nicht-fermentierbare Materialvolumen der Behälter auch nicht zur Erzeugung von Biogas bei, sondern verringert das mögliche Nutzvolumen in der Fermentationskammer. Weiterhin wird auch dadurch, dass die Behälter vorteilhaft nicht direkten Kontakt zu den Wänden der Fermentationskammer haben, sowie dadurch, dass die Behälter übereinander gestapelt werden und sich entsprechend ungenutzte Zwischenräume ergeben, das Nutzvolumen der Fermentationskammer reduziert. Dennoch überwiegen die Vorteile für die intendierte Anwendung, insbesondere als Insellösung.

Um eine gleichmäßige und vorteilhafte Durchströmung des Perkolats zu erreichen, sind die Behälter vorzugsweise zumindest mit einem flüssigkeitsdurchlässigen Boden, beispielsweise in Form eines Gitters, Geflechts oder Siebes, ausgestattet, so dass von oben in den obersten Behälter eingebrachtes Perkolat durch sämtliche Behälter hindurch strömen kann.

Ein weiterer Vorteil der Verwendung von stapelbaren Behältern zur Aufnahme des organischen Feststoffes ist darin zu sehen, dass die Gesamtkonstruktion der Fermentationskammern gegenüber den bekannten Fermentationskammern deutlich vereinfacht werden kann. Insbesondere können die Wände der Fermentationskammern nun weniger massiv ausgeführt werden und im extremen Fall beispielsweise lediglich durch eine Folie ausgebildet werden, da die Wände selbst nicht mit dem organischen Feststoff in Kontakt kommen und entsprechend keine statische Funktion oder Stützfunktion haben müssen. Die Wände der Fermentationskammern müssen hier lediglich gasdicht ausgeführt sein.

Gleiches gilt auch für die Decke der Fermentationskammer, welche in der hier vorgeschlagenen Lösung ebenfalls lediglich gasdicht ausgeführt werden muss, also beispielsweise auch als Folie ausgebildet sein kann, da eine mechanische Beanspruchung, beispielsweise durch das Anstoßen eines Baggerarmes oder anderen schweren Gerätes, ausgeschlossen ist, da die Fermentationskammern manuell mit den jeweiligen Behältern beschickt werden.

Hieraus ergibt sich ein gegenüber den bekannten Fermentationskammern deutlich vereinfachter Aufbau der Fermentationskammern, welcher auch in entlegenen Gebieten mit relativ einfachen Mitteln und insbesondere ohne großen logistischen bzw. infrastrukturellen Aufwand durchgeführt werden kann.

In einer bevorzugten Ausführungsform umfasst die Fermentationskammer Führungsmittel zum Führen des Behälters und/oder zur Definition eines Behälterstandortes in der Fermentationskammer. Die Führungsmittel können als Schienen, Rillen und/oder Vertiefungen zum Führen des mindestens einen Behälters und/oder zur Definition des Behälterstandortes ausgebildet sein. Auf diese Weise können die Behälter in eine entsprechende Position gebracht werden bzw. die möglichen Positionen der Behälter begrenzen. Insbesondere kann hierdurch durch eine entsprechende Anordnung verhindert werden, dass die Behälter mit den jeweiligen Wänden der Fermentationskammer in Berührung kommen, wodurch, wie bereits oben erläutert, eine sehr einfache und statisch nicht aufwändige Konstruktion der Wände der Fermentationskammer verwendet werden kann.

In einer weiteren bevorzugten Ausführungsvariante ist mindestens eine Perkolatdüse über jedem Behälter und/oder jedem möglichen Behälterstandort in der Fermentationskammer vorgesehen. Dadurch, dass die Perkolatdüsen jeweils genau über einem Behälterstandort angeordnet sind und dieser Behälterstandort klar definiert ist, kann eine optimale Verteilung des Perkolats in die jeweiligen organischen Feststoffe, welche in den Behältern vorgesehen sind, erfolgen. Hierdurch kann das gesamte Volumen der organischen Feststoffe zuverlässig mit Perkolat durchströmt werden. Eventuelle "tote Bereiche", welche beispielsweise im Randbereich der herkömmlichen Fermentationskammern auftreten können, können auf die genannte Weise vermieden werden.

Die Vorrichtung umfasst bevorzugt mindestens zwei Fermentationskammern derart, dass eine Beladung der jeweiligen Fermentationskammer abwechselnd, bzw. nacheinander mit dem jeweiligen gestarteten Fermentationsprozess durchgeführt werden kann. Abhängig von der jeweiligen Verweildauer bzw. der Prozessdauer des jeweiligen Vergärungsprozesses und dem Anfall von organischen Feststoffen kann eine entsprechende Anzahl an Fermentationskammern gewählt werden.

Die Größe einer Fermentationskammer liegt bevorzugt zwischen 5 und 45m³, bevorzugter zwischen 10 und 35 m³. Es handelt sich bei der Vorrichtung in dieser bevorzugten Ausführungsform entsprechend um eine Kleinanlage, welche als Insellösung in abgelegenen Gebieten verwendet werden kann und in welcher eine Kapazität von ca. 500t Biomasse pro Jahr verarbeiten kann.

Um den Flächenverbrauch der Vorrichtung zu reduzieren, kann bevorzugt der Perkolatbehälter, welcher das den organischen Feststoff durchströmende Perkolat enthält und dieses nach der Durchströmung auch wieder sammelt, unterhalb der Fermentationskammern vorgesehen sein. Dies kann beispielsweise in Form eines Erdtanks durchgeführt werden.

Die Speicherung des erzeugten Biogases kann beispielsweise mittels eines über der jeweiligen Fermentationskammer liegenden Gasspeichers, beispielsweise in Form eines beschichteten Textilsackes, vorgenommen werden.

Um bei einer Anlage Komponenten einsparen zu können und entsprechend die Kosten und logistischen Herausforderungen zu reduzieren, kann die Perkolatpumpe, welche zur Beschickung der Perkolatdüsen zum Beschicken des organischen Materials mit Perkolat alternierend auch zur Umwälzung des Perkolats in dem Perkolatbehälter verwendet werden. Auf diese Weise kann auf eine zusätzliche Pumpe bzw. auf ein zusätzliches Rührwerk zum Umwälzen des Perkolats im Perkolatbehälter verzichtet werden.

Die Größe der Anlage kann beispielsweise so ausgelegt sein, dass pro Jahr ca. 500 t organischer Feststoffe verarbeitet werden können. Eine solche Vorrichtung hat dann beispielsweise eine elektrische Anschlussleistung von ca. 20 kW bis 25 kW.

Bevorzugt ist die Anlage autark ausgelegt, also ein Anschluss an eine externe Stromversorgung nicht notwendig. Dies kann beispielsweise durch entsprechende Energiepuffer erreicht werden, welche zur Umwälzung des Perkolats herangezogen werden können. In einer bevorzugten Variante wird die Vorrichtung mit einer regenerativen Energiequelle gekoppelt, beispielsweise mit einer Windenergieanlage, einem Akkupuffer, einer Solarthermieanlage und/oder einer PhotovoltaikAnlage, welche die entsprechende benötigte Aktivierungsenergie bzw. Energie zum Betrieb der Perkolatpumpe bereitstellt.

Bevorzugt wird das Perkolat auf den organischen Feststoff aufgeströmt.

Um weitere Komponenten einzusparen, wird für die gesamte Vorrichtung, insbesondere auch bei Vorsehen mehrerer Fermentationskammern, lediglich ein einziges mobiles Gebläse oder aber ein Kamin zur Belüftung der Fermentationskammer nach Abschluss des Fermentationsverfahrens verwendet. Hierbei wird beispielsweise darauf geachtet, dass das mobile Gebläse an einem an der Fermentationskammer angebrachten Stutzen angeflanscht werden kann und nur dann mit Energie versorgt wird, wenn sowohl die Perkolatzufuhr zu der Fermentationskammer als auch der Gasabzug geschlossen sind. Das mobile Gebläse kann dabei bevorzugt mit einem Kamin zum Abblasen der aus der Fermentationskammer abgezogenen Gase versehen sein.

In einer vorteilhaften Weiterbildung ist eine Heizvorrichtung zum Heizen des Perkolats vorgesehen, bevorzugt mittels eines Wärmetauschers, welcher die Abwärme eines Blockheizkraftwerkes zum Heizen des Perkolats verwendet, oder bevorzugt mittels einer solarthermischen Heizvorrichtung.

Die oben genannte Aufgabe wird weiterhin durch ein Verfahren mit den Merkmalen des Anspruchs 14 gelöst. Vorteilhafte Ausgestaltungen des Verfahrens ergeben sich aus den Unteransprüchen.

Entsprechend umfasst das Verfahren zur Fermentation organischer Feststoffe das Einbringen des organischen Feststoffs in eine Fermentationskammer und das Aufbringen von Perkolat auf den in die Fermentationskammer eingebrachten organischen Feststoff. Erfindungsgemäß wird der organische Feststoff in mindestens einem Behälter in die Fermentationskammer eingebracht und in diesem Behälter in der Fermentationskammer fermentiert.

Durch das Einbringen und Fermentieren des organischen Feststoffes in dem Behälter kann eine deutlich vereinfachte Handhabung der Biomasse und des Gärrestes erreicht werden.

Bevorzugt wird der mindestens eine Behälter beim Einbringen in die Fermentationskammer mittels mindestens einem Führungsmittel an einen Behälterstandort in der Fermentationskammer geführt, wobei das Führungsmittel bevorzugt Schienen, Rillen und/oder Vertiefungen zum Führen des mindestens einen Behälters und/oder zur Definition des Behälterstandortes umfasst. Auf diese Weise kann bereits beim Einbringen der organischen Feststoffe ein Kontakt mit den Wänden der Fermentationskammer vermieden werden, so dass auf diese Weise eine vereinfachte Konstruktion der Wände der Fermentationskammer verwendet werden kann. Darüber hinaus kann durch eine definierte Platzierung der Behälter an vorgegebenen Orten das gezielte und effiziente Aufbringen von Perkolat unterstützt werden.

Entsprechend bevorzugt wird auf jeden Behälter und/oder jeden Behälterstandort mittels einer darüber angeordneten Perkolatdüse Perkolat aufgebracht.

In einer Weiterbildung des Verfahrens werden mindestens zwei Behälter in der Fermentationskammer übereinander gestapelt und die Fermentation wird in allen übereinander gestapelten Behältern gleichzeitig durchgeführt. Auf diese Weise kann der sich aus der Verwendung der Behälter ergebende Nachteil des verringerten Nutzvolumens teilweise ausgeglichen werden.

### Kurze Beschreibung der Figuren

Bevorzugte weitere Ausführungsformen und Aspekte der vorliegenden Erfindung werden durch die nachfolgende Beschreibung der Figuren näher erläutert.
- Figur 1: zeigt eine schematische perspektivische Ansicht einer Vorrichtung zur Fermentation organischer Feststoffe, wobei in der Ansicht die Decke über zwei der fünf Fermentationskammern fortgelassen wurde;
- Figur 2: zeigt eine weitere schematische perspektivische Darstellung der Vorrichtung aus Figur 1, mit weiteren Komponenten;
- Figur 3: zeigt eine schematische perspektivische Ansicht der Vorrichtung aus den Figuren 1 und 2 in einer schematischen Außenansicht;
- Figur 4: zeigt eine schematische perspektivische Darstellung des im Kellergeschoss angeordneten Perkolatbehälters der Vorrichtung aus den Figuren 1 bis 3;
- Figur 5: zeigt eine weitere schematische Darstellung des Perkolatbehälters der Vorrichtung gemäß den Figuren 1 bis 4; und
- Figur 6: zeigt die Rückseite der Vorrichtung in einer schematischen perspektivischen Darstellung.

### Detaillierte Beschreibung bevorzugter Ausführungsbeispiele

Im Folgenden werden bevorzugte Ausführungsbeispiele anhand der Figuren beschrieben. Dabei werden gleiche, ähnliche oder gleichwirkende Elemente in den unterschiedlichen Figuren mit identischen Bezugszeichen bezeichnet und auf eine wiederholte Beschreibung dieser Elemente wird teilweise verzichtet, um Redundanzen in der Beschreibung zu vermeiden.

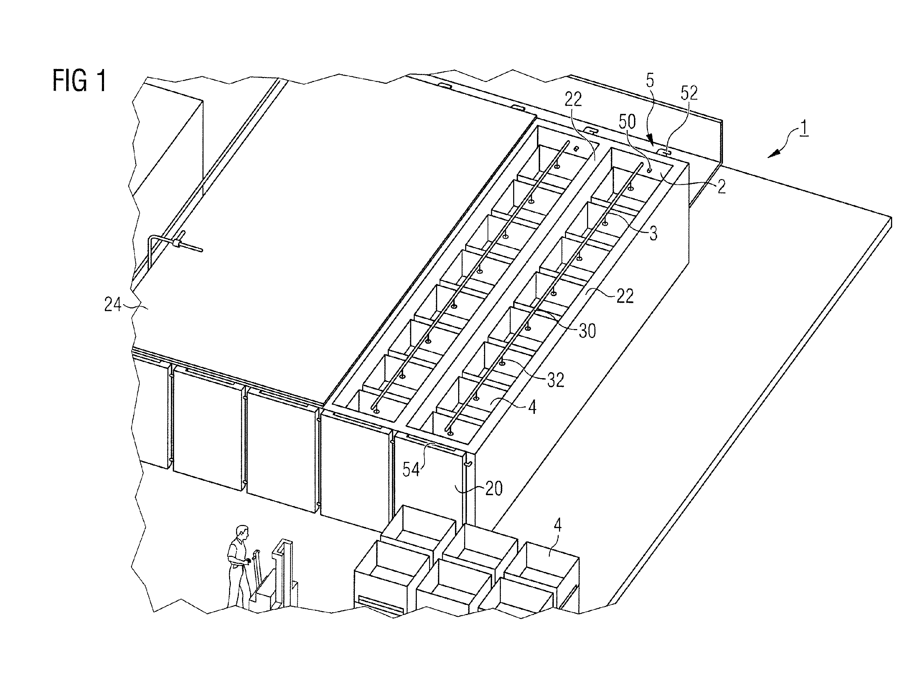

Figur 1 zeigt schematisch in einer perspektivischen Darstellung eine Vorrichtung 1 zur Fermentation organischer Feststoffe, welche fünf parallel zueinander und direkt nebeneinander angeordnete Fermentationskammern 2 zum Fermentieren organischer Feststoffe umfasst. Weiterhin ist eine Perkolationsvorrichtung 3 in Form der in jeder Fermentationskammer 2 angeordneten Perkolatleitung 30 und den aus der Perkolatleitung 30 austretenden Perkolatdüsen 32 vorgesehen.

In der Fermentationskammer 2 sind Behälter 4 vorgesehen, welche zur Aufnahme der organischen Feststoffe in der Fermentationskammer 2 dienen.

Unter dem Begriff "organische Feststoffe" wird hier zumindest stapelbare Biomasse wie beispielsweise Biomüll, biogene Abfallstoffe, Ernterückstände, Landschaftspflegegrün und/oder nachwachsende Rohstoffe verstanden. Auch Hausmüll in einer ungetrennten bzw. unsortierten Variante kann einen großen Anteil an Biomasse enthalten, welche als organischer Feststoff dienen kann.

Wie sich aus Figur 1 sofort ergibt, sind die Behälter 4 an festen Orten in der Fermentationskammer 2 angeordnet, so dass sich hier eine feste Struktur bzw. ein festes Muster innerhalb der Fermentationskammer 2 ergibt. Über jedem Behälter 4 in der gezeigten Ausführungsform ist entsprechend eine Perkolatdüse 32 angeordnet, so dass der jeweilige Behälter 4 optimal mit Perkolat beaufschlagt werden kann. Auch wenn mindestens zwei Behälter 4 übereinander gestapelt sind, kann auf diese Weise sicher gestellt werden, dass auch der untere Behälter ausreichend mit Perkolat beaufschlagt wird.

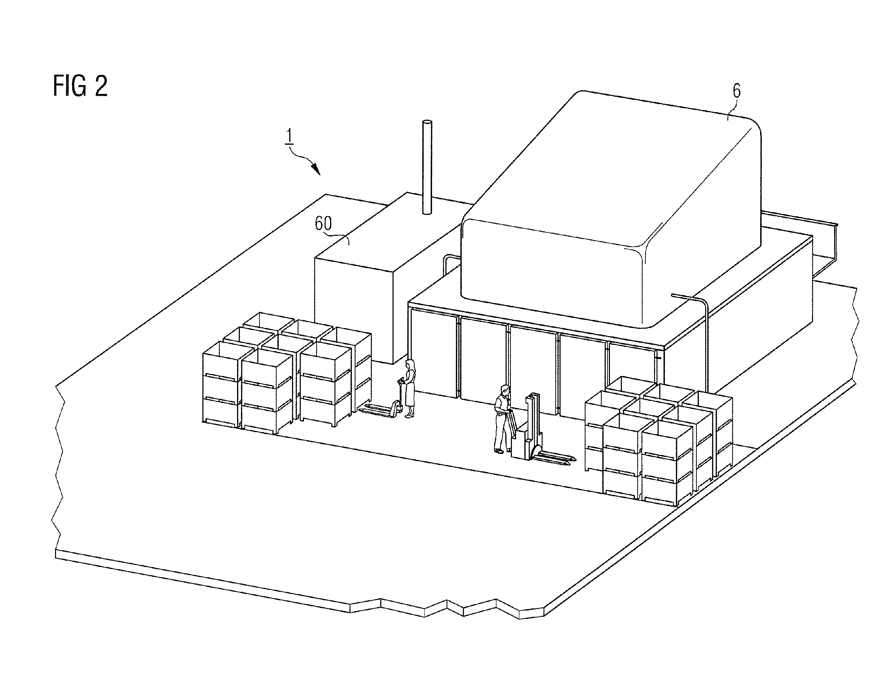

In dem in Figur 1 gezeigten Ausführungsbeispiel umfasst die Vorrichtung 1 zur Fermentation organischer Feststoffe fünf nebeneinander und parallel zueinander angeordnete Fermentationskammern 2, welche jeweils zur Aufnahme der Behälter 4 vorgesehen sind und entsprechend dimensioniert sind. In dem gezeigten Ausführungsbeispiel haben die Fermentationskammern 2 jeweils eine Größe von ca. 30 m³. Die Fermentationskammern 2 sind auf ihrer zur Beladung der Fermentationskammern 2 vorgesehenen Vorderseite mit einer Türe 20 gasdicht verschlossen. Die jeweiligen Wände 22 und die Decke 24 der Fermentationskammer 2 sind ebenfalls gasdicht ausgebildet. Das beim Fermentationsprozess erzeugte Biogas wird über entsprechende Rohrleitungen abgeführt und in einem beispielsweise in Figur 2 gezeigten Blockheizkraftwerk 2 zu Strom und Wärme umgewandelt.

Die Behälter 4 begrenzen den Raum bzw. das Volumen, in welchem der organische Feststoff aufgenommen werden kann. Die Behälter 4 dienen auch zum Beladen der Fermentationskammer 2 mit organischen Feststoffen, sowie zum Entladen der Fermentationskammer 4.

Durch die Verwendung der Behälter 4 kommt der organische Feststoff, welcher in den Fermentationskammern 2 aufgenommen wird, nicht mit den Wänden 22, mit der Tür 20 und/oder mit der Decke 24 der Fermentationskammer 2 in Kontakt. Entsprechend können die Wände 22, die Decke 24 und/oder die Tür 20 so ausgebildet sein, dass sie lediglich ihre Funktion als gasdichter Abschluss ausbilden, jedoch keine Stützfunktion für den organischen Feststoff aufweisen. Diese Stützleistung wird nun von den jeweiligen Wänden der Behälter 4 übernommen. Entsprechend kommt der organische Feststoff nicht in Berührung mit den Wänden, den Decken und/oder der Tür, so dass diese Komponenten der Fermentationskammer 2 anders dimensioniert werden können.

Die Behälter 4 sind, wie sich beispielsweise aus Figur 1 ergibt, in einem festgelegten Muster in den Fermentationskammern 2 aufgenommen. Dieses festgelegte Muster kann beispielsweise dadurch erreicht werden, dass in den Fermentationskammern 2 hier nicht gezeigte Führungsmittel vorgesehen sind. Diese Führungsmittel können beispielsweise in Form von Führungsschienen, Führungsrillen, Aufnahmevertiefungen und/oder auch Aufnahmen für an der Unterseite der Behälter 4 vorgesehene Füße/Zapfen sein, so dass die jeweilige Stellposition der Behälter 4 festgelegt ist.

Entsprechend werden die organischen Feststoffe in die Fermentationskammern 2 mittels der gezeigten Behälter 4 eingebracht. Damit kann die Handhabung der organischen Feststoffe beim Einbringen bzw. beim Austragen des Gärrestes erheblich gegenüber den bereits bekannten Fermentationsvorrichtungen erleichtert werden. Insbesondere können die Behälter 4 so dimensioniert werden, dass sie entweder getragen werden können, oder aber mit leichten Maschinen, wie beispielsweise einem handbetriebenen Hubstapler oder einem Hubwagen in die jeweilige Fermentationskammer 2 eingebracht werden können. Gleichermaßen können die Behälter 4 mit dem Gärrest aus den jeweiligen Fermentationskammern 2 einfach ausgetragen werden und unter Umständen direkt als Kompost bzw. Dünger auf entsprechenden Feldern verteilt werden.

Die Behälter 4 sind so ausgebildet, dass sie zum einen übereinander stapelbar sind um eine handhabbare Größe der Behälter sicherzustellen, und zum anderen weisen sie einen flüssigkeitsdurchlässigen Boden auf, beispielsweise in Form eines Geflechts, eines Siebbodens und/oder eines Gitters, derart, dass das über die Perkolatdüsen 32 eingebrachte Perkolat von dem oberen Behälter 4 in den/die darunter liegenden Behälter fließen kann.

In Figur 1 ist weiterhin schematisch eine Belüftungsvorrichtung 5 angedeutet, welche einen entsprechenden Belüftungsstutzen 50 aufweist, welcher in die Fermentationskammer 2 hineinragt. Die Belüftungsvorrichtung 5 weist einen entsprechenden Flansch 52 auf der Außenseite der Fermentationskammer 2 auf, wobei dieser Flansch 52 dazu dient, eine mobile Belüftungsvorrichtung anzuflanschen. Um die Fermentationskammer 2 entsprechend nach Abschluss des Fermentationsvorganges zu belüften und so ein gefahrloses Entladen der Fermentationskammer 2 zu ermöglichen, wird eine Belüftungsöffnung 54 in der Türe 20 geöffnet und die mobile Belüftungseinheit an den Flansch 52 angeflanscht. Auf diese Weise wird Frischluft von der Öffnung 54 zum Anschlussstutzen 50 hin gesaugt. Durch eine entsprechende Spülung wird hier erreicht, dass die Fermentationskammer 2 vollständig mit Umgebungsluft befüllt ist, so dass eine gefahrlose Entladung der Fermentationskammer 2 erreicht werden kann.

Der Betrieb der mobilen Belüftungsvorrichtung am Flansch 52 wird nur dann freigegeben, wenn sowohl die Perkolatzufuhr zu der jeweiligen Fermentationskammer 2, als auch der entsprechende Gasabzug für das Biogas aus der Fermentationskammer geschlossen sind.

In Figur 2 ist die in Figur 1 gezeigte Vorrichtung 1 in einer weiteren schematischen Darstellung gezeigt. Das in den jeweiligen Fermentationskammern 2 erzeugte Biogas wird in einem Gasspeicher 6 gespeichert. Der Gasspeicher 6 ist oberhalb der Fermentationskammern 2 angeordnet, um die benötigte Grundfläche zum Aufbau der Vorrichtung 1 möglichst gering zu halten.

Neben den Fermentationskammern 2 ist ein Blockheizkraftwerk 60 schematisch angedeutet, in welchem das erzeugte Biogas sofort in Strom und Wärme umgewandelt werden kann.

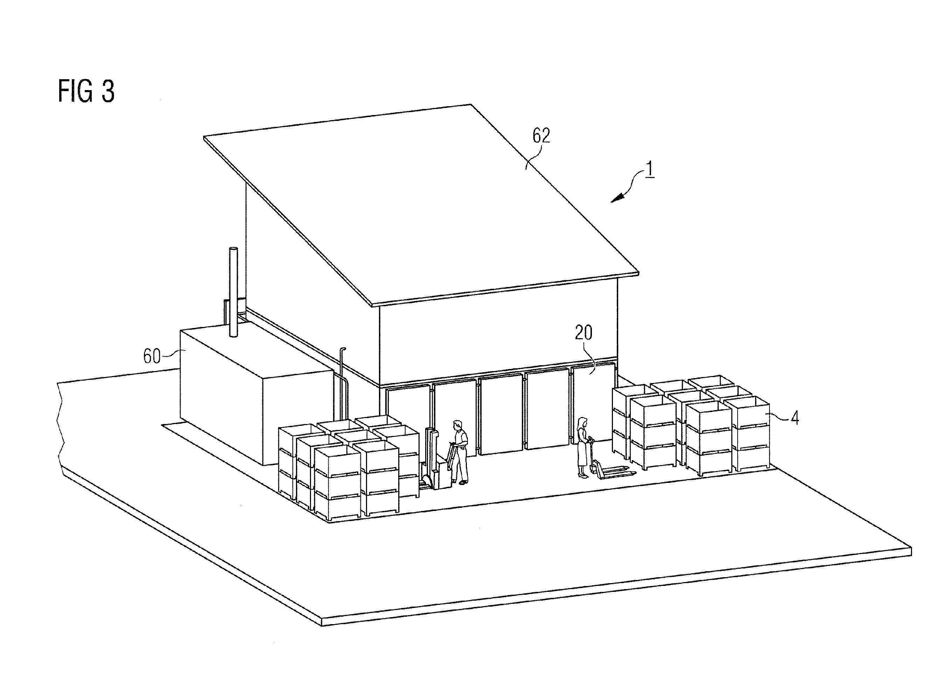

Figur 3 zeigt die Vorrichtung 1 in einer weiteren schematischen Ansicht, wobei nun der Gassack von einer entsprechenden Dachkonstruktion 62 überdacht ist, so dass die gesamte Anlage überschaubar und in sich abgeschlossen präsentiert ist. Die Vorrichtung in der in Figur 3 gezeigten Dimension ist beispielsweise dafür geeignet, bis zu 500 t organische Feststoffe pro Jahr zu verarbeiten. Das entsprechende Blockheizkraftwerk, welches hier angeschlossen ist, hat eine elektrische Leistung von ungefähr 20 bis 25 kW.

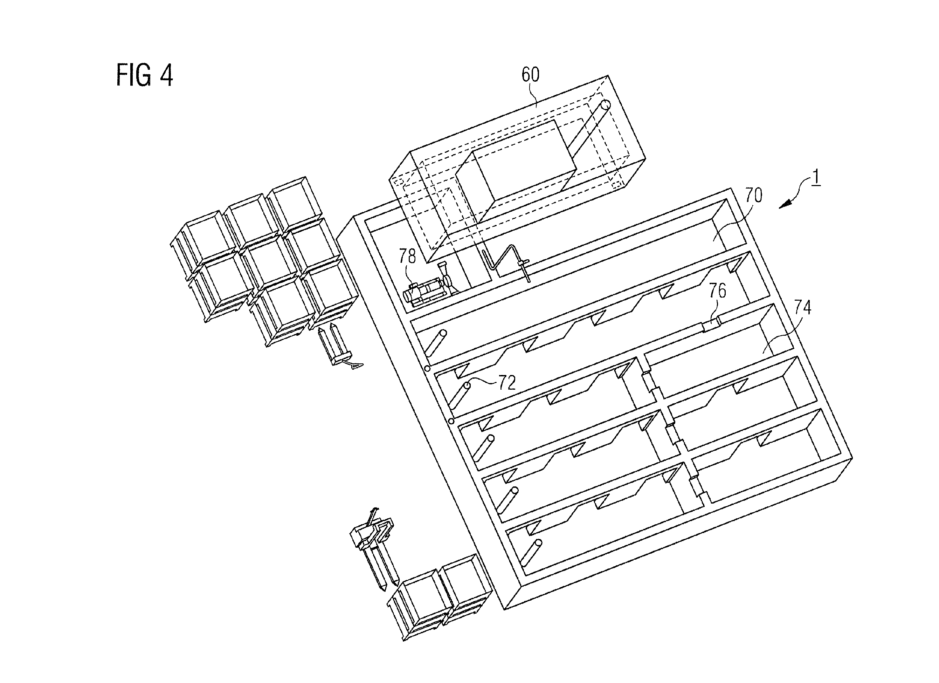

Figur 4 zeigt die Anlage 1 in einer schematischen Draufsicht, wobei hier hauptsächlich das Untergeschoss der Vorrichtung 1 gezeigt ist. Im Untergeschoss ist ein Perkolatbehälter 70 vorgesehen, welcher über entsprechende Zuläufe 72 mit den direkt darüber liegenden Fermentationskammern 2 verbunden ist. Das Perkolat, welches über die in Figur 1 gezeigten Perkolatdüsen 32 durch den organischen Feststoff hindurch strömt, strömt dann entlang des Bodens der Fermentationskammer und hin zum Abfluss 72, so dass das Perkolat wieder in den Perkolatbehälter 70 zurückgeführt wird, nachdem es den organischen Feststoff durchflossen hat. Die Anordnung des Abflusses 72 ist so gewählt, dass hier ein gasdichter Abschluss erreicht wird, beispielsweise durch Bildung eines Siphons.

Ein abgetrennter Bereich 74 des Perkolatbehälters 70 wird als Endlager verwendet, derart, dass überschüssiges Perkolat über eine Überschwemmschwelle 76 in das Endlager 74 eingeschwemmt wird.

Eine Perkolatpumpe 78 ist in einem separaten Raum vorgesehen, wobei die Perkolatpumpe 78 zum einen dazu dient, den organischen Feststoff in den Fermentierungskammern 2 entsprechend über die in Figur 1 gezeigten Perkolatdüsen 32 mit Perkolat zu beaufschlagen. Zum anderen dient die Perkolatpumpe 78 dazu, das Perkolat im Perkolatbehälter 70 zu zirkulieren bzw. zu bewegen.

Da der Schritt des Perkolierens der organischen Feststoffe in der jeweiligen Fermentationskammer 2 üblicherweise nur wenige Minuten pro Stunde dauert, kann die Perkolatpumpe 78 die übrige Zeit zur Umwälzung des Perkolats im Perkolatbehälter 70 verwendet werden. Durch diese doppelte Funktion der Perkolatpumpe 78 kann hier zumindest eine Komponente eingespart werden.

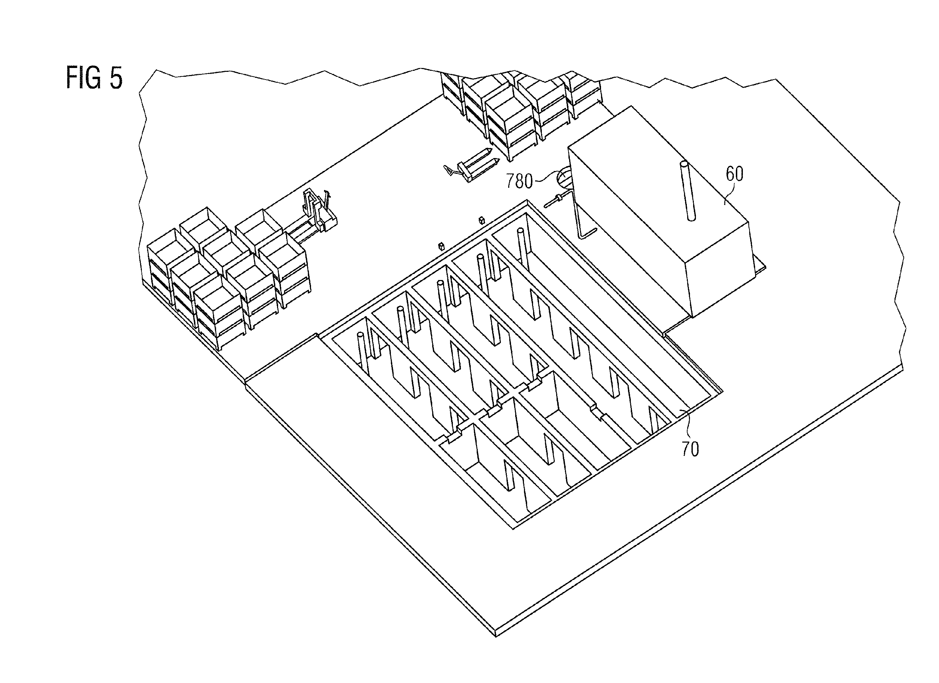

Figur 5 zeigt schematisch noch einmal das Kellergeschoss bzw. Untergeschoss der Vorrichtung, so wie es beispielsweise auch in Figur 4 gezeigt ist.

Der Einstieg in den Raum der Perkolatpumpe 78 kann über ein Mannloch 780 erfolgen, so dass auch der Aufbau des Kellergeschosses sowie der Zugang hierzu sehr einfach gestaltet werden kann.

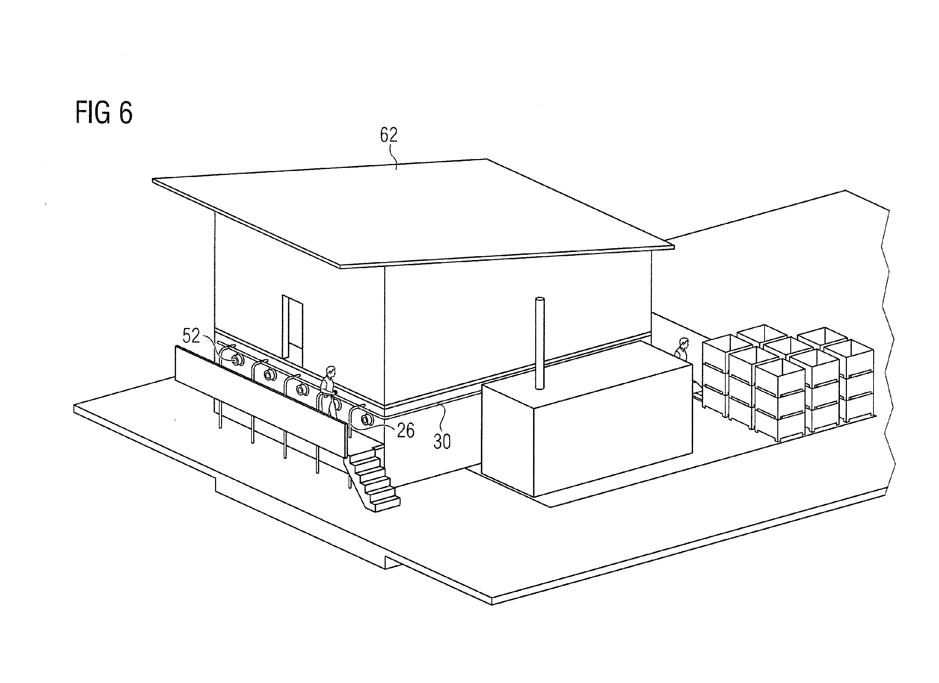

Figur 6 zeigt die in den vorhergehenden Figuren gezeigte Anlage noch einmal von der anderen Gebäudeseite aus. Hier verlaufen die jeweiligen Anschlüsse für die in den Fermentationskammern 2 verlaufenden Perkolatleitungen 30 sowie die Gasableitungen 26 zur Ableitung des Biogases aus den Fermentationskammern. Der Flansch 52 zum Anflanschen des mobilen Gebläses zur Belüftung der Fermentationskammern vor dem Öffnen und Entladen ist ebenfalls zu erkennen.

Mittels einer bevorzugten Schaltung kann das mobile Gebläse an dem Flansch 52 nur dann betrieben werden, wenn sowohl die Perkolatleitung 30 als auch die Gasleitung 26 geschlossen sind.

Die in den Figuren gezeigte Vorrichtung 1 ist als Kleinanlage konzipiert, welche auch als Insellösung betrieben werden kann.

Bevorzugt kann dazu die Dachkonstruktion 62 mit entsprechenden Solarzellen bzw. photovoltaischen Zellen belegt werden, um ein vollkommen autarkes Energiezentrum bereitzustellen. Über die Energie der photovoltaischen Zellen kann beispielsweise auch die Perkolatpumpe 78 betrieben werden wenn das Blockheizkraftwerk 60 nicht betrieben wird.

Der beispielsweise in Figur 2 gezeigte Gasspeicher 6 dient dabei auch als Reserve zur Speicherung von Energie. In abgelegenen Gebieten muss das Blockheizkraftwerk 60 beispielsweise nicht kontinuierlich betrieben werden, sondern kann nur dann betrieben werden, wenn Energie tatsächlich benötigt wird, also von der Dämmerung an bis in die Nacht hinein. Die übrige Zeit könnte das Blockheizkraftwerk 60 auch abgeschaltet sein. In dieser Variante ist das Blockheizkraftwerk bevorzugt so dimensioniert, dass es eine relativ größere Anschlussleistung aufweist, als die entsprechend kontinuierlich im Fermentationsprozess abgegebene Leistung.

Die Abwärme des Blockheizkraftwerkes 60 kann zur Beheizung des Perkolats in dem Perkolatbehälter 70 dienen. In einer Variante wird das Perkolat in dem Perkolatbehälter 70 mittels einer separaten Heizvorrichtung beheizt. Hierfür eignen sich sowohl elektrische Heizvorrichtungen oder gasbetriebene Heizvorrichtungen, sowie solarthermisch betriebene Heizvorrichtungen.

In einer Variante kann das Blockheizkraftwerk 60 kontinuierlich betrieben werden und die erzeugte elektrische Energie wird in einem Akkumulatorspeicher zwischengespeichert. Auf diese Weise kann die Lebensdauer des Blockheizkraftwerkes aufgrund des kontinuierlichen Betriebes verbessert werden.

Um einen besonders einfachen Deckenaufbau der in Figur 1 gezeigten Decke 24 über den jeweiligen Fermenterkammern 2 zu erreichen, kann hier eine Bretterkonstruktion gewählt werden, welche lediglich mit einer gasdichten Folie belegt ist. Eine entsprechende Konstruktion kann auch für die Wände 22 der Fermenterkammern 2 gewählt werden, da die organischen Feststoffe in den Behältern 4 aufgenommen werden und entsprechend eine Stützfunktion der Seitenwände nicht notwendig ist.

Das Volumen der jeweiligen Fermentationskammern 2 ist an das jeweilige Aufkommen an organischen Feststoffen in dem durch die Anzahl der Fermentationskammern 2 gewählten Zeitintervall angepasst. Bei fünf Kammern und einer mittleren Fermentationsdauer von 4 Wochen findet der Fermentationsvorgang immer in vier Kammern gleichzeitig statt und eine der Kammern kann während der Woche mit neuer Biomasse beladen werden kann. Entsprechend muss eine Kammer den Anfall von organischem Feststoff einer Woche aufnehmen können.

Soweit anwendbar, können alle einzelnen Merkmale, die in den einzelnen Ausführungsbeispielen dargestellt sind, miteinander kombiniert und/oder ausgetauscht werden, ohne den Bereich der Erfindung zu verlassen.

## Patentansprüche

1. Vorrichtung (1) zur Fermentation organischer Feststoffe, umfassend mindestens eine Fermentationskammer (2) zum Fermentieren organischer Feststoffe und mindestens eine Perkolationsvorrichtung (3) zum Aufbringen von Perkolat auf organische Feststoffe in der Fermentationskammer (2),
**dadurch gekennzeichnet, dass**
mindestens ein Behälter (4) zum Aufnehmen der organischen Feststoffe in der Fermentationskammer (2) vorgesehen ist.

2. Vorrichtung gemäß Anspruch 1, wobei die Fermentationskammer (2) Führungsmittel zum Führen des mindestens einen Behälters (4) und/oder zur Definition eines Behälterstandortes in der Fermentationskammer (2) umfasst, bevorzugt Schienen, Rillen und/oder Vertiefungen zum Führen des mindestens einen Behälters und/oder zur Definition des Behälterstandortes.

3. Vorrichtung gemäß Anspruch 1 oder 2, wobei eine Perkolatdüse (32) über jedem Behälter und/oder über jedem Behälterstandort in der Fermentationskammer (2) vorgesehen ist.

4. Vorrichtung gemäß einem der vorstehenden Ansprüche, wobei eine Fermentationskammer (2) ein Volumen zwischen 5 und 40m³, bevorzugt zwischen 12 und 32 m³, aufweist.

5. Vorrichtung gemäß einem der vorstehenden Ansprüche, wobei mindestens zwei Fermentationskammern (2) vorgesehen sind.

6. Vorrichtung gemäß einem der vorstehenden Ansprüche, wobei mindestens ein Perkolatbehälter (70) zum Sammeln und Speichern des Perkolats unter einer Fermentationskammer (2) angeordnet ist.

7. Vorrichtung gemäß Anspruch 6, wobei eine Perkolatpumpe (78) zum Aufbringen des Perkolats aus dem Perkolatbehälter (70) vorgesehen ist, und die Perkolatpumpe (78) auch zur Umwälzung des Perkolats in dem Perkolatbehälter (70) eingerichtet ist.

8. Vorrichtung gemäß einem der vorstehenden Ansprüche, wobei ein Gasspeicher (6) zum Speichern des bei der Fermentation in der Fermentationskammer (2) erzeugten Biogases vorgesehen ist, wobei der Gasspeicher (6) über der Fermentationskammer (2) angeordnet ist und wobei der Gasspeicher bevorzugt in Form eines beschichteten Textilsackes vorgesehen ist.

9. Vorrichtung gemäß einem der vorstehenden Ansprüche, wobei ein Belüftungsstutzen (50) zur Belüftung der Fermentationskammer (2) vorgesehen ist, bevorzugt zur Belüftung mittels eines mobilen Gebläses und/oder eines Kamins, welche an die Fermentationskammer (2) von außen anbindbar sind.

10. Vorrichtung gemäß einem der vorstehenden Ansprüche, wobei mindestens ein Behälter (4) einen flüssigkeitsdurchlässigen Boden aufweist, bevorzugt in Form eines Siebbodens, eines Geflechtes, eines Gitters etc.

11. Vorrichtung gemäß einem der vorstehenden Ansprüche, wobei mindestens ein Behälter (4) stapelbar ist.

12. Vorrichtung gemäß einem der vorstehenden Ansprüche, wobei die Vorrichtung autark betreibbar ist, bevorzugt durch Kopplung mit einer Windenergieanlage, einem Akkupuffer, einer Solarthermieanlage und/oder einer Photovoltaikanlage.

13. Vorrichtung gemäß einem der vorstehenden Ansprüche, wobei eine Heizvorrichtung zum Heizen des Perkolats vorgesehen ist, bevorzugt mittels eines Wärmetauschers, welcher die Abwärme eines Blockheizkraftwerkes zum Heizen des Perkolats verwendet, oder bevorzugt mittels einer solarthermischen Heizvorrichtung.

14. Verfahren zur Fermentation organischer Feststoffe, umfassend das Einbringen des organischen Feststoffs in eine Fermentationskammer (2) und das Aufbringen von Perkolat auf den in die Fermentationskammer (2) eingebrachten organischen Feststoff,
**dadurch gekennzeichnet, dass**
der organische Feststoff in mindestens einem Behälter (4) in die Fermentationskammer eingebracht wird und in diesem Behälter (4) in der Fermentationskammer fermentiert wird.

15. Verfahren gemäß Anspruch 14, wobei der mindestens eine Behälter (4) beim Einbringen in die Fermentationskammer (2) mittels mindestens einem Führungsmittel an einen Behälterstandort in der Fermentationskammer (2) geführt wird, wobei das Führungsmittel bevorzugt Schienen, Rillen und/oder Vertiefungen zum Führen des mindestens einen Behälters und/oder zur Definition des Behälterstandortes umfasst.

16. Verfahren gemäß Anspruch 14 oder 15, wobei auf jeden Behälter (4) und/oder jeden Behälterstandort mittels einer darüber angeordneten Perkolatdüse (32) Perkolat aufgebracht wird.

17. Verfahren gemäß einem der Ansprüche 14 bis 16, wobei mindestens zwei Behälter (4) in der Fermentationskammer (2) übereinander gestapelt werden und die Fermentation in allen übereinander gestapelten Behältern (4) gleichzeitig durchgeführt wird.

18. Verfahren gemäß einem der Ansprüche 14 bis 17, wobei das Verfahren autark betrieben wird, bevorzugt durch Zufuhr von Energie aus der Kopplung mit einer Windenergieanlage, einem Akkupuffer, einer Solarthermieanlage und/oder einer Photovoltaikanlage.
